(19) Europäisches Patentamt
European Patent Office
Office européen des brevets

(11) EP 1 068 870 B1

(12) # EUROPEAN PATENT SPECIFICATION

(45) Date of publication and mention of the grant of the patent:
**07.06.2006 Bulletin 2006/23**

(21) Application number: **99910779.0**

(22) Date of filing: **29.03.1999**

(51) Int Cl.:
***A61K 33/24*** *(2006.01)* ***A61K 31/165*** *(2006.01)*
***A61K 31/275*** *(2006.01)* ***A61K 31/28*** *(2006.01)*
***A61K 31/395*** *(2006.01)* ***A61P 35/00*** *(2006.01)*
***A61K 45/06*** *(2006.01)* *A61K 45/06 (2006.01)*
*A61K 33/24 (2006.01)* *A61K 45/06 (2006.01)*
*A61K 31/28 (2006.01)* *A61K 33/24 (2006.01)*
*A61K 31/165 (2006.01)*

(86) International application number:
**PCT/JP1999/001633**

(87) International publication number:
**WO 1999/051246 (14.10.1999 Gazette 1999/41)**

(54) **ANTITUMOR AGENTS**
ANTITUMORALE MITTEL
AGENTS ANTITUMORAUX

(84) Designated Contracting States:
**AT BE CH CY DE DK ES FI FR GB GR IE IT LI LU MC NL PT SE**

(30) Priority: **03.04.1998 JP 10870898**
**14.08.1998 JP 22984398**

(43) Date of publication of application:
**17.01.2001 Bulletin 2001/03**

(73) Proprietor: **Ajinomoto Co., Inc.**
**Tokyo 104-8315 (JP)**

(72) Inventors:
- **MORINAGA, Yoshihiro**
**Ajinomoto Co., Inc.**
**Kawasaki-shi**
**Kanagawa-ken 210-0801 (JP)**
- **NIHEI, Yukio**
**Ajinomoto Co., Inc.**
**Kawasaki-shi**
**Kanagawa-ken 210-0801 (JP)**
- **SUGA, Yasuyo**
**Ajinomoto Co., Inc.**
**Kawasaki-shi**
**Kanagawa-ken 210-0801 (JP)**
- **SUZUKI, Manabu**
**Ajinomoto Co., Inc.**
**Kawasaki-shi**
**Kanagawa-ken 210-0801 (JP)**
- **OHISHI, Kazuo**
**Ajinomoto Co., Inc.**
**Kawasaki-shi**
**Kanagawa-ken 210-0801 (JP)**
- **OKANO, Akira**
**Ajinomoto Co., Inc.**
**Kawasaki-shi**
**Kanagawa-ken 210-0801 (JP)**

(74) Representative: **Nicholls, Kathryn Margaret et al**
**Mewburn Ellis LLP**
**York House**
**23 Kingsway**
**London WC2B 6HP (GB)**

(56) References cited:
**EP-A- 0 276 051** **EP-A- 0 641 767**
**WO-A-00/48590** **WO-A-01/12579**
**WO-A-92/16486** **WO-A-95/00129**
**WO-A-95/20960** **WO-A-97/08184**
**WO-A-02/056692** **US-A- 4 935 450**
**US-A- 5 700 826**

- **NAKATA ET AL: "Synergistic interaction between cisplatin and tamoxifen delays the emergence of cisplatin resistance in head an neck cancer cell lines" CANCER CHEMOTHERAPY AND PHARMACOLOGY, vol. 35, no. 6, 1995, pages 511-518, XP008010359**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 068 870 B1

- MCCLAY, E.F. ET AL: "Tamoxifen delays the development of resistance to cisplatin in human melanoma and ovarian cancer cell lines" BRITISH JOURNAL OF CANCER (1994), 70(3), 449-52 , XP008010360
- PATENT ABSTRACTS OF JAPAN vol. 005, no. 180 (C-079), 19 November 1981 (1981-11-19) & JP 56 103192 A (KITANI YOSHINORI), 18 August 1981 (1981-08-18)
- CUSHMAN M ET AL: "SYNTHESIS AND EVALUATION OF STILBENE AND DIHYDROSTILBENE DERIVATIVES AS POTENTIAL ANTICANCER AGENTS THAT INHIBIT TUBULIN POLYMERIZATION" JOURNAL OF MEDICINAL CHEMISTRY, AMERICAN CHEMICAL SOCIETY. WASHINGTON, US, vol. 34, no. 8, 1 August 1991 (1991-08-01), pages 2579-2588, XP000571676 ISSN: 0022-2623
- WOODS J A ET AL: "THE INTERACTION WITH TUBULIN OF A SERIES OF STILBENES BASED ON COMBRETASTATIN A-4" BRITISH JOURNAL OF CANCER, LONDON, GB, vol. 71, no. 4, 1995, pages 705-711, XP000978556 ISSN: 0007-0920
- GANN, Vol. 69, No. 3, June 1978, (China), MITSUTARO AKAO and KEIKO KURODA, " Enhancing and Inhibitory Effects of Some Stilbene and Steroid Compounds on Induction of Hepatoma in Rats Fed 3'-Methyl-4-(Dimethylamino)Azobenzene", p. 375-382, XP002926541
- PHYTOCHEMISTRY, Vol. 33, No. 4, 1993, ERKKI MANNILA et al., "Anti-Leukaemic Compounds Derived from Stilbenes in Picea Abies Bark", p. 813-816, XP002926542

## Description

### TECHNICAL FIELD

This invention relates to a novel antitumor agent. More particularly, it relates to an antitumor agent comprising a stilbene derivative and a platinum coordination (coordinate) compound, such as Cisplatin, for example a chemotherapeutic drug (chemotherapy drug; chemotherapeutic agent) against cancers, to an antitumor agent comprising a stilbene derivative or a platinum coordination compound used therefor, and to uses of these effective ingredients for the treatment (therapy), suppression, amelioration (improvement) of tumors.

### BACKGROUND ART

Nowadays, a wide variety of chemotherapeutic agents are used for treatment, suppression and prevention of tumors, especially malignant solid tumors. Although these agents may have the tumor reducing effect, however , it is not possible for these known agents to release the pain of the patient from the tumors, due to acquisition of resistance against the agent or relapse of the tumors. Therefore, it is desired at present to develop further superior antitumor agents.

Despite stilbene derivatives, having cis-stilbene as a fundamental skeleton, are known to exhibit strong mitosis inhibitory activities and cytotoxity, these stilbene derivatives are as yet not available as a pharmaceutical agent because of their low solubility in water.

Recently, stilbene derivatives having the activity for inhibiting tubulin polymerization, especially phosphorylated pro-drug of Combretastatin-A4 (See US Patent 56122), having improved water solubility, and stilbene derivatives by the present Assignee (Japanese Patent Kokai Publications JP-A-7-228558 and JP-A-8-301831) have been proposed as a drug, and clinical use of these stilbene derivatives is felt to be promising.

On the other hand, it is desired to improve the efficacy of these stilbene derivatives further, in the aspects of antitumor activities and safety.

### OBJECT AND PROBLEM OF THE INVENTION

It is an object of the present invention to develop a superior antitumor agent, specifically, to develop a pharmaceutical preparation capable of improving the efficacy of a stilbene derivative and, in particular, to develop and provide an antitumor agent exhibiting superior efficacy in both the antitumor activity and in safety in curing (treating) malignant tumors.

### DISCLOSURE OF INVENTION

For overcoming the above problem and achieving the object, the present inventors have conducted perseverant researches, and have found that a stilbene derivative of formula (1), administered together with a platinum coordination compound, such as Cisplatin, exhibits synergistic therapeutic effects to improve the tumor enhancement (tumor growth) inhibiting activities of the platinum coordination compound effectively, however, the effect of the stilbene derivative is superior to those of other chemotherapeutic drugs and the platinum coordination compounds, such as Cisplatin, so that the stilbene derivative is a chemotherapeutic drug higher in efficacy to patients. That is, by employing the stilbene derivatives in combination with platinum coordination compound, usable as the chemotherapeutic agents, especially Cisplatin, as a chemotherapeutic drug used in treating solid cancers, antitumor effects as well as safety may be exhibited synergistically towards fulfilment of the above-mentioned object and requirement from the problem. This finding has led to completion of the present invention.

The process of arriving at the present invention is now explained in more detail.

For finding a composition of a chemotherapeutic drug against cancer with higher efficacy, as may be demonstrated by improved efficacy against tumor grafted to a mouse, in connection with the composition containing Cisplatin as a chemotherpeutic drug used for curing (treating) various solid cancers (tumors), such as pulmonary cancer (lung cancer), the present inventors have conducted researches, using, as an index for achieving more promising clinical efficacy, the complete curing effect for mouse tumor and freeness from augmentative body weight decreasing action.

As a result of these researches, the present inventors have found that, by combining a stilbene derivative, having preferably in-vitro tubulin polymerization inhibiting activities with a platinum coordination compound, it is possible to achieve a synergistic tumor complete curing effect and freeness from augmentative body weight decreasing action.

Among currently known therapeutic agents, having the tubulin polymerization inhibiting activities as the main action, there are Vincristine, Vindesine, Vinblastine, etc. These agents are used for curing solid cancers in a multi-drug (polypharmacy) therapy, including Cisplatin, etc., in expectation of augmentation of the clinical efficacy. However, in simultaneous administration of Vindesine and Cisplatin against mouse tumor, used for the present test, no tumor complete

curing effect, as the reference for efficacy, has not been noted. Therefore, the efficacy, that is the tumor complete curing effect, of the antitumor agent of the present invention, is thought to be the inherent (proper) action in the antitumor agent of the present invention.

As for the safety (toxicity), no augmentative body weight decreasing effect, as may be noted in the composition containing Vindesine and Cisplatin, is noted in the composition containing the compound shown by the following structural formula (3):

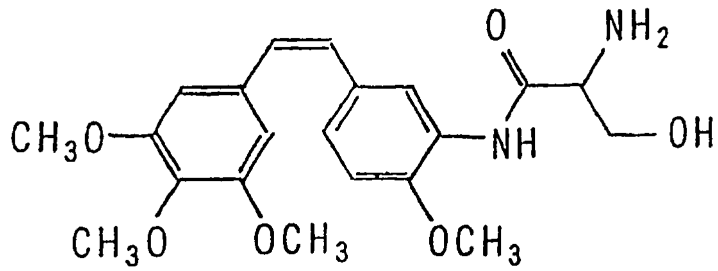

(3)

and Cisplatin.

From the above results, it has been clarified that the composition of the present invention is appreciably higher in efficacy for animals, especially human beings (patients), than the composition containing Cisplatin or solely other chemotherapeutic drug, and represents an antitumor agent, especially a cancer chemotherapy agent, possibly relieving the patient from the pains of tumor. The present invention has been completed on the basis of these findings.

That is, the present invention is promising as providing a novel antitumor agent, for example, a chemotherapeutic drug against cancer (cancer chemotherapy agent), comprising simultaneously or separately two types of active ingredients, namely a stilbene derivative and a platinum coordination compound, such as Cisplatin.

EMBODIMENTS OF INVENTION

The present invention is directed to an antitumor agent characterized by comprising a stilbene derivative of formula (1) and a platinum coordination compound.

The present invention also encompasses such a stilbene derivative and a platinum coordination compound combined as the two sorts of pharmaceutical preparations separately comprising these two ingredients. The present invention also encompasses, as an antitumor agent or an assistant antitumor agent, such a pharmaceutical preparation comprising the stilbene derivative for use as the antitumor agent of the present invention or a pharmaceutical preparation comprising the platinum coordination compound for use as the antitumor agent of the present invention .

The tumor against which the antitumor agent of the present invention is administered and applied to the subject contains all sorts of tumors occurring in an animal, especially in a human being. Preferably, the antitumor agent of the present invention may be used for inhibiting proliferation of tumor cells of a human being. The antitumor agent of the present invention is a pharmaceutical preparation aimed at curing (treatment) and suppression, prevention of the tumors.

There is no particular limitation to the form of administration of the antitumor agent. The platinum coordination compound is routinely administered parenterally, whilst the stilbene derivative is presently scheduled to be administered parenterally. The present invention encompasses an antitumor agent consisting in the combination of the two having distinct forms of administration.

As the stilbene derivative used in the present invention, a compound with cis-stilbene as a fundamental skeleton and which exhibits in-vitro tubulin polymerization inhibiting activity and/or an antitumor activity of formula (1) is used. As the antitumor activity, tumor cell proliferation inhibiting activity is preferred. Any suitable pharmaceutically allowable salts, solvates (solvation products) such as hydrates thereof, may be used as the stilbene derivatives in the present invention, provided that the derivatives exhibit such objective antitumor activity when used in vivo.

As stilbene derivatives, having the cis-stilbene as a fundamental skeleton, there are compounds represented by the following general formula (1) :

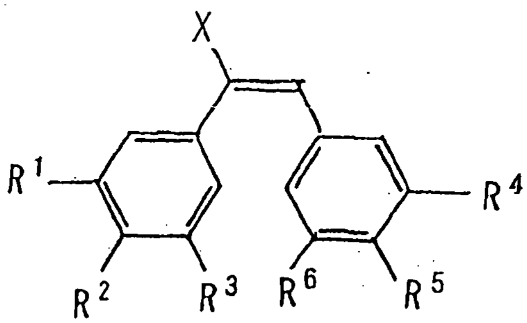

(1)

and corresponding salts, hydrates and solvates (solvation products), and especially pharmaceutically acceptable forms thereof.

In the above formula $R^1$, $R^2$ and $R^3$ independently denote lower alkoxy groups, $R^4$, $R^5$ and $R^6$ independently denote any substituent of a hydrogen atom, a halogen atom (fluorine, chlorine atoms, etc.), a nitro group, a lower alkoxy group, a phosphoric acid amide (a substituent formed on phosphoric acid amidation with an amino group: $-NHPO_3H_2$, hereinafter the same), an amino lower alkoxy group, a lower alkyl amino lower alkoxy group, a di-lower alkyl amino lower alkoxy group, a mercapto group, a lower alkyl thio group, an amino group, a lower alkyl amino group, a di-lower alkyl amino group, a lower alkyl group, an amino lower alkyl group, a trifluoro methyl group, a lower alkanoyl group, a lower alkanoyl amino group and an amino acid acylamino group and X denotes a hydrogen atom or a nitrile group.

The number of carbon atoms in the above described lower alkyl group and the lower alkoxy group is 1 to 5, respectively while that of the lower alkanoyl group is 2 to 6.

The amino acid acyl group in the amino acid acylamino group is an acyl group derived from the amino acid. The amino acids may be enumerated by $\alpha$-amino acids, $\beta$-amino acids and $\gamma$-amino acids. Examples of preferred amino acids include glycine, alanine, leucine, serine, lysine, glutamic acid, asparatic acid, threonine, valine, isoleucine, ornithine, glutamine, asparagine, tyrosine, phenylalanine, cysteine, methionine, arginine, $\beta$-alanine, tryptophan, proline, histidine, etc. In particular, threonine and serine are preferred in view of pharmaceutical effects and safety. Although any one of these amino acids may be of the L-, D- or DL-form, the L-form is preferred.

As described above, the stilbene derivative in the present invention is a compound having a cis-stilbene skeleton in its structure and which exhibits a tubulin polymerization inhibiting activity and/or an antitumor activity. The stilbene derivative may be exemplified specifically by, for example, tumor proliferation inhibitive stilbene derivative, disclosed in prior art publications, such as the patent publication ( See U.S. Patent Nos. 4,996,237, 5,561,122 and 5,430,062, Japanese Patent Kokai Publications JP-A-7-228558, JP-A-8-301831 and JP-A-10-81673, corresponding to Japanese patent Application Serial No. 236603/1996 filed by the present Applicant on September 6, 1996.). The prior art stilbene derivatives, described in these patent publications, can be used for the stilbene derivatives of the present invention, insofar as the prior art stilbene derivatives are in meeting with the above definition for the stilbene derivatives in the present invention.

The above mentioned stilbene derivatives may be manufactured by the routine technique including the method disclosed in the above mentioned known publications. It is noted that stilbene derivative to be developed in future may be manufactured and used for the present invention in the same manner as described above.

Among the stilbene derivatives of the present invention, there are salts of stilbene.

As the stilbene derivative used in the present invention, a compound represented by the following general formula (1):

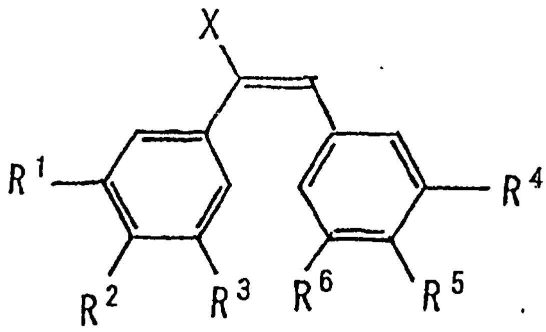

(1)

is more preferred. In the above fromula (1), $R^1$, $R^2$, $R^3$ and $R^5$ denote a methoxy group, $R^4$ denotes an amino group or an amino acid acylamino group and $R^6$ and X denote a hydrogen atom.

Among the compounds represented by the above general formula (1), a compound represented by the following general formula (3), which may be also referred to as a compound (3) below:

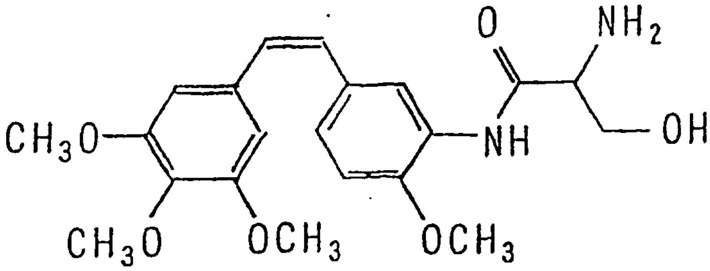

(3)

is particularly preferred. The compound (3) is named as (Z)-1-(3-amino-4-methoxyphenyl)-2-(3,4,5-trimethoxyphenyl)-ethene-L- serine amide, and is soluble in water. The compound (3) may be in the form of a salt exemplified by hydrochloride, acetate, methanesulfonate and the like.

The manufacture of the compound (3), which may be in the form of the pharmaceutically acceptable salts, hydrates and solvates, and the manufacture of oral and/and parenteral pharmaceutical composition containing the above compound (3), its inert pharmaceutically acceptable carrier(s) and/or diluent(s), are extensively disclosed in Japanese Patent Kokai Publication JP-A-8-301831.

The platinum coordination compound used in the present invention is such a compound which gives a platinum preferably in an ionic form, and is also a compound exhibiting antitumor activity, more preferably a platinum coordination compound exhibiting tumor cell proliferation preventative (inhibiting) properties.

Specified examples of the platinum coordination compounds, employed in the present invention, preferably include Cisplatin, cis-diamminediacoplatinum (II)-ion, chloro (diethylene triamine)-platinum (II) chloride, dichloro (ethylene diamine)- platinum (II), diammine(1, 1-cyclobutane dicarboxylate) platinum (II) (carboplatin), spiroplatin, iproplatin, diammine (2-ethylmalonate)-platinum (II), ethylene diammine malonate platinum (II), aqua (1, 2-diaminodichlohexane)-sulfate platinum (II), (1, 2-diaminocyclohexane) malonate platinum (II), (4-caloxyphthalate)-(1, 2-diaminocyclohexane) platinum (II), (1, 2-diaminocyclohexane)-(isocitrate) platinum (II), (1, 2-diaminocyclohexane)-cis (pyruvate) platinum (II), (1, 2-diaminocyclohexane)-oxalate platinum (II), Ormaplatin and tetraplatin.

Certain explanations will be hereinafter made as to a platinum complex, included in the platinum coordination compound according to the present invention, as a chemotherapeutic drug.

Cisplatin or cis-dichlorodiammine platinum (II) has been successfully used for long as a chemotherapeutic drug in the therapy of various malignant tumors in the human being.

More recently, other diamino-platinum complexes also have shown efficacy as the chemotherapeutic drugs in curing various malignant tumors in the human being. These diamino-platinum complexes may be enumerated by, for

example, spiroplatinum and carboplatinum.

Cisplatin and other diamino-platinum complexes have widely been used as the chemotherapeutic drugs in the human being. However, these are not therapeutically efficient for all patients or all sorts of tumors. In expectation of possible increase in efficacy, numerous attempts have been made towards using Cisplatin in combination with Vindesine by Garalla et al. (Garalla, R. J. et al., Ann. Intern. Med., 95, 414-420, 1980) or using Cisplatin in combination with VP-16 by Congeval et al. (Congeval, E. et al., Cancer, 51, 2751-2756, 1982). Although such combined application has achieved certain improvement in the efficacy ratio, it cannot be said that the pains of the patients by these tumors may be completely relieved by these measures.

As the platinum coordination compounds used in the present invention, there are those compounds already known as the chemotherapeutic drugs (See Tamura, T. et al., Jpn J. Clin. Oncol. vol.18 (1). 27 (1988) and Fukuda, M. et al., Cancer Chemother. Pharmacol., vol.26,393 (1990).).

It is necessary to increase the efficacy of the tumor proliferation suppressing activity of Cisplatin and other diamino-platinum complexes in order to relieve the patient of the pain from the tumors.

Among the platinum coordination compounds used for the present invention, Cisplatin, Carboplatin and Nedaplatin are more desirable in their curative effects. Although the compound "Cisplatin", which means cis-dichloro diamine platinum (II), may be manufactured by prior art techniques, it may also be available commercially. For example, Cisplatin may be procured, as a powder for constituting with water, an aseptic physiological saline water or other suitable excipient, under the trade name of Platinol (Registered Trademark) from Bristol Myers-Squibb Co. or under the trade name of "randa Inj." from NIPPON KAYAKU CO., LTD.

Most of other platinum coordination compounds used in the present invention are commercially availabe or may be manufactured by known or routinely used techniques.

When the antitumor agent of the present invention is to be used, stilbene derivatives in an amount sufficient to inhibit tumor proliferation may be combined with platinum coordination compounds, and administered to the subject, an animal, especially a human being, in need of curing, alleviation or prevention of tumors, especially a human being suffering from proliferation of tumor cells, to inhibit the proliferation of tumor cells in the human being.

In this case as described above, the two types of such efficacious ingredients in the present invention may be contained in combination in a pharmaceutical preparation, and however, such an each combined pharmaceutical preparation obtained on combining two different preparations containing one of the two type of efficacious ingredients in the present invention to realize the objective antitumor agent, is encompassed by the present invention. Moreover, a pharmaceutical preparation containing one of the two efficacious ingredients is also encompassed by the present invention, if such pharmaceutical preparation has the objective of being used in combination with the pharmaceutical preparation containing the other efficacious ingredient in the present invention.

One of the preferred embodiments in the present invention is to use the compound (3) in an amount effective to inhibit proliferation of tumor cells in combination with cisplatin to inhibit proliferation of tumor cells.

The inhibition of proliferation of tumor cells means inhibition of proliferation of the tumor cells sensitive to therapy including administration of an effective amount of the stilbene derivatives, such as the compound (3), and the platinum coordination compounds, such as cisplatin, to e.g., a human being suffering from proliferation of tumor cells.

In an acceptable case, this administration suppresses proliferation of tumor cells or diminishes the measurable size of the tumors. In an optimum case, the tumor undergoes regression completely.

As described above, there is no particular limitation to the method of administering the antitumor agent of the present invention to the human being, such that it may be administered orally or parenterally, such as by intravenous, subcutaneous or intramuscular route. For prompt efficacy, parenteral administration, such as by intravenous and subcutaneous administration, by infusion, etc. is preferred. In the method for administering the pharmaceutical preparation according to the present invention, the stilbene derivative may be administered simultaneously with the platinum coordination compound or the two may be sequentially administered in an optional order. The practically desirable method and sequence for administration are varied depending on the individual preparation of the stilbene derivative used, such as the compound (3), individual preparation of the platinum coordination compound in use, such as Cisplatin, individual tumor cells being cured, and the individual hosts being treated. The optimum method and sequence for administration of the stilbene derivative and the platinum coordination compound under preset given conditions may be suitably selected by those skilled in the art with the aid of the routine technique and the information contained in the present specification.

An efficacious tumor proliferation inhibiting amount of the stilbene derivative and the platinum coordination compound means a curative unit inhibiting proliferation of the tumor cells sensitive to administration in the human being suffering from proliferation of tumor cells. The practically desirable curative unit is varied depending on the individual dosage forms of the stilbene derivative used, such as the compound (3), individual dosage forms of the platinum coordination compound uses, such as Cisplatin, individual tumor cells being cured and the individual hosts being treated. The optimum curative units for preset given conditions may be suitably selected by those skilled in the art with the aid of the curative test units and the information contained in the present specification.

When administering the antitumor agent of the present invention, the administration schedule for the platinum

coordination compound is preferably determined by setting approximately 1 to 500 $mg/m^2$ of the body surface area per curative unit as a reference. When using Cisplatin and the compound (3), these may be administered simultaneously, Cisplatin is administered prior to administration of the compound (3)(later administration of the compound (3)), or the compound (3) is administered prior to administration of Cisplatin (later administration of Cisplatin). Alternatively, these methods may be used in combination. As for the preferred amount of administration of Cisplatin, approximately 10 to 100 $mg/m^2$ of the body surface area per day of Cisplatin is preferably administered simultaneously in curative units of the compound (3) each of continuous one to five days on end. The platinum coordination compound in an infusion form is preferably infused once or twice a week. This weekly infusion is preferably repeated several times insofar as the undesirable side effect actions such as nephrotoxicity and neurotoxicity do not give rise to a taboo. It is possible to use other conventionally used techniques simultaneously with the administration of the platinum compound, such as Cisplatin or the other platinum coordination compound.

The antitumor agent of the present invention is sufficient to be a pharmaceutical preparation comprising at least the stilbene derivative and the platinum coordination compound as described above, such that the two active ingredients may be contained as a mixture in the pharmaceutical preparation. However, the two active ingredients in the present invention may also be contained separately in distinct pharmaceutical preparations used in combination. It is noted that such a pharmaceutical preparation containing other agents (third and fourth medical ingredients and so on) such as other antitumor agents, may naturally be encompassed by the present invention, insofar as the effective ingredients used in the present invention are contained in the pharmaceutical preparation. Moreover, it is possible for carriers, diluents and other substances, pharmaceutically acceptable for any of the pharmaceutical preparations in the present invention (a sole pharmaceutical preparation containing both ingredients in the present invention and separate pharmaceutical preparations separately each containing one of the two ingredients for use in combination) to be contained in the antitumor agent of the present invention.

As the suitable pharmaceutically acceptable carriers and diluents, used in the antitumor agent of the present invention, those carriers etc known to those skilled in the art of preparation of pharmaceutical preparations, may be used as appropriate (See, for example, Japanese Patent Kokai Publication JP-A-8-301831 and the other aforementioned prior art publications.). The antitumor agent of the present invention may be suitably applied parenterally, as discussed above. In this case, the antitumor agent is prepared into an intravenous infusion or injection, along with pharmaceutically acceptable carriers by variable methods known to those skilled in the art. Preferably, the pharmaceutical agent is manufactured by a routine technique in e.g., a unit dosage form and in the form of a freeze-dried mixture of two effective ingredients, and is re-prepared in water or other suitable liquid infusion in administration.

The ratio of the two ingredients for the pharmaceutical preparation for the antitumor agent of the present invention may be varied in a wide range, depending on a number of factors, such as a desired amount of administration and on the pharmaceutically acceptable carrier in use. As for the amounts or combination in administering the stilbene derivative in the pharmaceutical preparation as the antitumor agent of the present invention, the stilbene derivative of approximately 0.01 to 1000 and, in particular, approximately 0.1 to 100 parts by weight of the stilbene derivative, to 1 part by weight of the platinum coordination compound present in the pharmaceutical preparation as the antitumor agent of the present invention, are preferably employed. So, when the pharmaceutical preparation in the present invention containing two active ingredients is to be administered to the patient, it is administered in an amount which will give the above-defined administration range.

If the pharmaceutical preparation is to be administered stepwise, the above-defined administration range can be set as the average ratio for the separate pharmaceutical preparations.

Preferably, 5 to 500 mg of the platinum coordination compound, more preferably 10 to 50 mg as Cisplatin, 0.1 to 10,000 mg of the stilbene derivative and more preferably 1 to 1,000 mg as the compound (3) may be contained for each dosage of the pharmaceutical preparation according to the present invention. It is desirable that mannitol and/or sodium chloride be contained in routine amounts in the pharmaceutical preparation of Cisplatin. The physiological pharmaceutical value of the pharmaceutical composition used as an injection or infusion liquid is suitably adjusted by the content of a buffer well-known in the art.

## PREFERRED EMBODIMENTS

The present invention is now explained in more detail with reference to preferred embodiments thereof. It is to be noted that these are given only as an example and are not intended to limit the invention

(Example 1) Antitumor Effect and Tests on Safety

(Preparation of Pharmaceutical preparation)

Using the following composition, a pharmaceutical preparation for infusion was prepared, whereby the compound (3) was used as the stilbene derivative.

compound (3) as hydrochloride: 10 mg
physiological saline water: 10 ml

As Cisplatin, a pharmaceutical preparation marketed by NIPPON KAYAKU CO., LTD. under the trade name of "randa Inj." (a preparation containing 0.5 mg of Cisplatin in 1 ml of a solution) was used.

(Test on Efficacy in Mice (Test on Antitumor Activity and Safety))

10 mg of a colonic tumor of mouse, colon 26 was inoculated under the skin of the back of CDF1 mice (day 0). After one week, the tumor was measured to calculate the volume of the tumor and the mice were classified into several groups (each group ;n = 5). Injection of the pharmaceutical preparation was started. The compound (3) ( 1 mg of hydrochloride in 1 ml of physiological saline water) and cisplatin ( 0.5 mg of Cisplatin in 1 ml of "randa Inj.") were bolusly injected under the skin of the back and into the tail vein on the 7th, 11th and 15th days.

The individual in which the tumor has not been ascertained by palpation on the 60th day was deemed to have its tumor cured completely. The results are shown in Table 1.

The "VP-16" is a chemotherapeutic drug which is often used clinically in combination with Cisplatin. [+CDDP] means that Cisplatin was administered in an amount of 5 mg/kg/day and [-CDDP] means that Cisplatin was not administered at all. [n.d] means that the test was not carried out. Meanwhile, the dosages of Cisplatin, VP-16 and Vindesine represent the maximum dosage without death due to the toxicity in the administration schedule in the present example.

By the following equation, the rate of body weight change at the 21st day was calculated. The results are shown in Table 2.

Rate of body weight change (%) = [{(body weight - weight of the tumor) at the 21st day} - {(body weight - weight of the tumor) at the 7th day }]/{(body weight - weight of the tumor) at the 7th day} x 100

[Table 1] test on antitumor activity (1)

| Sample | Amount of Administration | Number of instances of complete regression of tumors | |
|---|---|---|---|
| | mg/kg/day | -CDDP | +CDDP |
| Control | - | 0/5 | 0/5 |
| Compound (3) | 5 | n.d. | 1/5 |
| Compound (3) | 10 | 0/5 | 4/5 |
| Vindesine | 2 | n.d. | 0/5 |
| VP-16 | 30 | 0/5 | 0/5 |

[Table 2] test on safety (1)

| Sample | Amount of Administration | Rate of Body Weight Change | |
|---|---|---|---|
| | mg/kg/day | -CDDP | +CDDP |
| Control | - | -12.0 | -11.1 |
| Compound (3) | 5 | n.d. | -4.2 |
| Compound (3) | 10 | -7,7 | -1,9 |
| Vindesine Vindesine | 2 | n.d. | -23.2 |
| VP-16 | 30 | -1.0 | -9.9 |

As clearly shown in the results of table 1, the antitumor agent of the present invention, that is the combination of the stilbene derivative and the platinum coordination compound, was improved as to synergistic pharmaceutical activity, especially in the effect of complete regression of tumors , as compared to a pharmaceutical preparation composed of any one of two ingredients.

On the other hand, in the pharmaceutical preparation, used clinically in combination with Cisplatin, such as Vindesine or VP-16, the complete regression of tumor was not observed.

Concerning the safety aspect, undesirable loss of body weight, such as is noted in the combination of Vindesine or VP-16 with the platinum coordination compound, was not observed in the combination of the two ingredients of the present invention, as clearly shown in the results of Table 2.

(Example 2) Antitumor Effect and Test on Safety 2

(Preparation of Pharmaceutical preparation)

Pharmaceutical preparations for infusion were prepared in accordance with the following composition using the compounds (4) and (5), shown by the following chemical formulas as the stilbene derivatives:

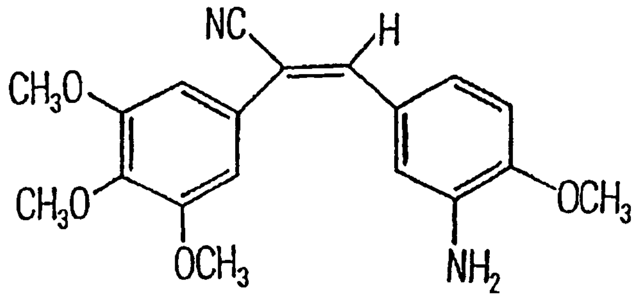

(4)

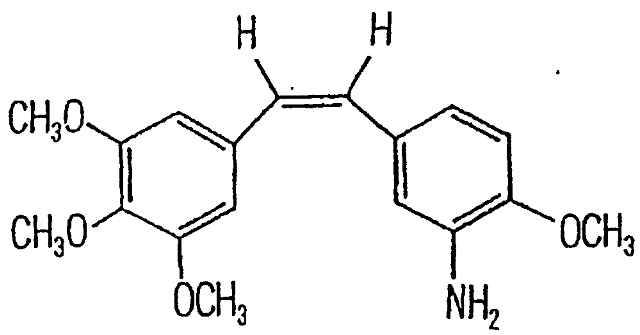

(5)

| | |
|---|---|
| compound (4) (as hydrochloride) | 5 mg; |
| Tween 80 | 0.5 ml; and |
| physiological saline water | 9.5 ml. |
| compound (5)(as hydrochloride) | 10 mg; |
| Tween 80 | 0.5 ml; and |
| physiological saline water | 9.5 ml. |

As in Example 1 as described above, the pharmaceutical preparation ["randa Inj."] marketed by NIPPON

KAYAKU CO., LTD. (containing 0.5 mg of Cisplatin in 1 ml solution) and the pharmaceutical preparation ["paraplatin injection"] marketed by Bristol Myers-Squibb Co. (containing 10 mg of Carboplatin in 1 ml) were used as the Cisplatin and as Carboplatin, respectively.

(Test on Efficacy in Mice [Test on Antitumor Activity and Safety]

10 mg of a colonic tumor of mouse, colon 26 was inoculated under the skin of the back of CDF1 mice (day 0). After one week, the tumor was measured to calculate the volume of the tumor and the mice were classified into several groups (each group ;n = 5). Injection of the pharmaceutical preparation was started.

The compounds (3) to (5), Cisplatin and Carboplatin were injected as bolus into the tail vein on the 7th, 11th and 15th days.

The individual in which the tumor has not been ascertained by palpation on the 60th day was deemed to have its tumor cured completely. The results were shown in Tables 3 and 4.

[+CBDCA] and [+CDDP] means that 50 mg/kg/day of carboplatin and 5 mg/kg/day of cisplatin were administered, respectively, whilst [-CBDCA] and [-CDDP] means no administration of carboplatin and cisplatin. Meanwhile, the dosages of cisplatin and carboplatin denote the maximum dosages without death due to toxicity in the administration schedule in the present embodiment.

The rate of body weight change at the 21st day was calculated in accordance with the equation used in the Example 1. The results are shown in tables 5 and 6.

[Table 3] Test on Antitumor Activity (2)

| Sample | Amount of Administration | Number of instances of complete regression of tumors | |
|---|---|---|---|
| | mg/kg/day | -CBDCA | +CBDCA |
| Control | - | 0/6 | 0/6 |
| Compound (3) | 20 | 0/6 | 1/6 |

[Table 4] Test on Antitumor Activity (3)

| Sample | Amount of Administration | Number of instances of complete regression of tumors | |
|---|---|---|---|
| | mg/kg/day | -CDDP | +CDDP |
| Control | - | 0/6 | 0/6 |
| Compound (4) | 5 | 0/6 | 4/6 |
| Compound(5) | 20 | 0/6 | 6/6 |

[Table 5] Test on Safety (2)

| Sample | Amount of Administration | Rate of Body Weight Change | |
|---|---|---|---|
| | mg/kg/day | -CBDCA | +CBDCA |
| Control | - | -22.1 | -9.6 |
| Compound(3) | 20 | -7.4 | 1.8 |

[Table 6] Test on Safety (3)

| Sample | Amount of Administration | Rate of Body Weight Change | |
|---|---|---|---|
| | mg/kg/day | -CDDP | +CDDP |
| Control | - | -22.1 | -11.4 |
| Compound(4) | 5 | -7.0 | -13.9 |
| Compound (5) | 20 | -2.1 | -0.3 |

As clearly shown in the results of Tables 3 and 4, the antitumor agent of the present invention exhibits superior

antitumor activity by employing the stilbene derivative and the platinum coordination compound in combination.

Moreover, concerning the safety aspect, as clearly shown in the results of Tables 5 and 6, outstanding improvement may be noticed by employing the two ingredients of the present invention.

Finally, it may be seen from the results of Tables 1 to 6 that, with the combination of the stilbene derivative and the platinum coordination compound according to the present invention, the efficacy as the antitumor agent is improved synergistically beyond the expectation by those skilled in the art, such that a practically highly useful antitumor effect can be achieved.

EFFECTS OF INVENTION

The antitumor agent according to the present invention, comprising a stilbene derivative and a platinum coordination compound, such as Cisplatin, in combination or as a mixture, can be used as an antitumor agent, especially as a cancer chemotherapeutic drug (cancer chemotherapy drug; chemotherapeutic agent), which may be highly effective as curing (treatment), suppression and prevention of tumors, especially solid cancer (solid carcinoma), due to the synergistic effect derived from the combination of two types of the efficacious ingredients.

It is therefore possible to use these two types of the active ingredients for therapy (treatment), suppression, amelioration (improvement) of such tumors.

## Claims

1. An antitumor agent comprising a stilbene derivative and a platinum coordination compound, and optionally containing one or more of pharmaceutically acceptable carriers, diluents and other substances required for the pharmaceutical preparation wherein said stilbene derivative is at least one of compounds represented by the following general formula (1):

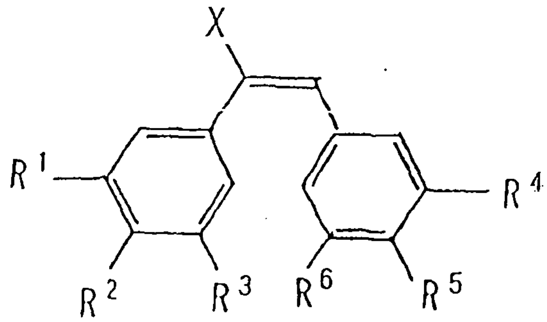

(1)

in which the compound may be in the form of salt, hydrate or solvate; in which $R^1$, $R^2$ and $R^3$ are independent from each other and each denotes a C1-C5 alkoxy group, $R^4$, $R^5$ and $R^6$ are independent from each other and each denotes any substituent group of a hydrogen atom, a halogen atom, a nitro group, a C1-C5 alkoxy group, a phosphoric acid amide, an amino C1-C5 alkoxy group, a C1-C5 alkyl amino C1-C5 alkoxy group, a di-C1-C5 alkyl amino C1-C5 alkoxy group, a mercapto group, a C1-C5 alkyl thio group, an amino group, a C1-C5 alkyl amino-group, a di-C1-C5 alkyl amino group, a C1-C5 alkyl group, an amino C1-C5 alkyl group, a trifluoromethyl group, a C2-C6 alkanoyl group, a C2-C6 alkanoyl amino group and an amino acid acylamino group, and X denotes a hydrogen atom or a nitrile group.

2. The antitumor agent as defined in claim 1, wherein said stilbene derivative is a compound having a cis-stilbene skeleton exhibiting in-vitro tubulin polymerization inhibiting activity and/or an antitumor activity; and said platinum coordination compound is a compound exhibiting antitumor activity.

3. The antitumor agent as defined in claim 1 or claim 2, wherein said platinum coordination compound is selected from Cisplatin, Carboplatin and Nedaplatin.

4. The antitumor agent as defined in any one of the preceding claims, wherein said stilbene derivative is represented by the following general formula (1), and said platinum coordination compound is a compound exhibiting an antitumor activity:

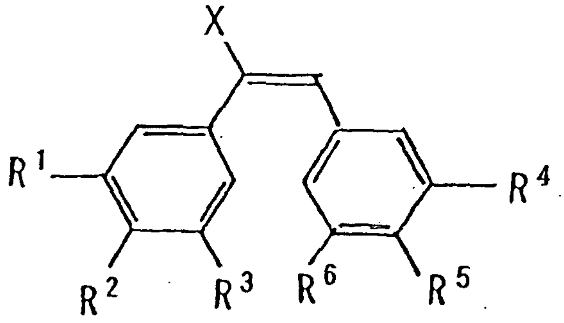

(1)

,in which said compound may be in the form of salt; and wherein $R^1$, $R^2$, $R^3$and $R^5$ denote a methoxy group, $R^4$ denotes any one substituent of an amino group and an amino acid acylamino group, and $R^6$ and X denote a hydrogen atom.

5. The antitumor agent as defined in claim 4, wherein said stilbene derivative is represented by The following structural formula (3), and said platinum coordination compound is a compound exhibiting an antitumor activity:

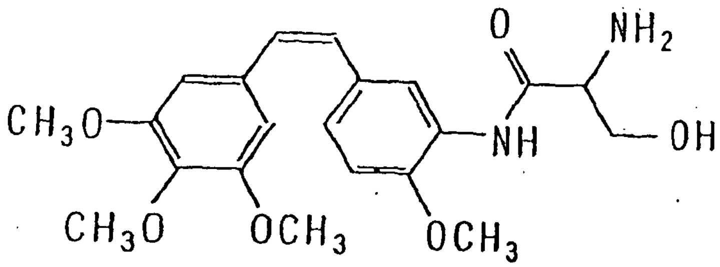

(3)

6. The antitumor agent according to any one of the preceding claims wherein said platinum coordination compound is Cisplatin, and said stilbene derivative is a compound represented by the following structural formula (3) :

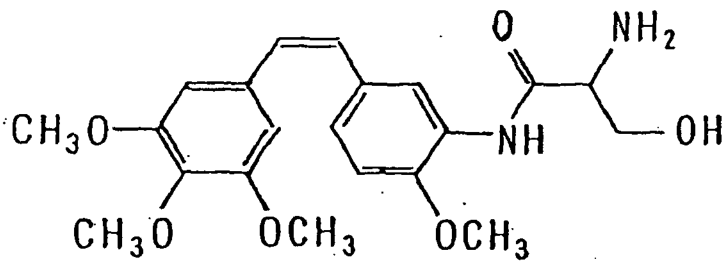

(3)

7. Use of a stilbene derivative of formula (1) as set out in claim 1 and a platinum coordination compoundin the preparation of an antitumor agent or of a medicament for treatment, suppression or amelioration of a tumor.

8. A stilbene derivative of formula (1) as set out in claim 1 in combination with a platinum coordination compound for simultaneous or separate use as an antitumor agent.

**Patentansprüche**

1. Antitumormittel, das ein Stilbenderivat und eine Platinkoordinationsverbindung und gegebenenfalls einen oder mehrere pharmazeutisch geeignete Träger, Verdünnungsmittel oder andere Substanzen enthält, die für die Arzneimittelzubereitung erforderlich sind, worin das Stilbenderivat mindestens eine Verbindung der folgenden allgemeinen Formel (1) ist:

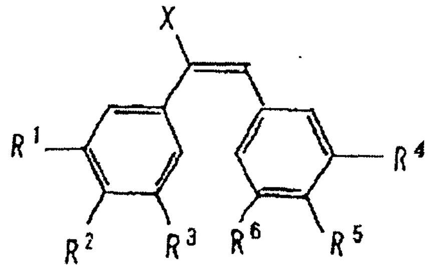

wobei die Verbindung in der Form eines Salzes, Hydrats oder Solvats sein kann; worin $R^1$, $R^2$ und $R^3$ unabhängig voneinander sind und jeweils eine $C_1$-$C_5$-Alkoxygruppe darstellen, $R^4$, $R^5$ und $R^6$ unabhängig voneinander sind und jeweils eine beliebige, unter einem Wasserstoffatom, einem Halogenatom, einer Nitrogruppe, einer $C_1$-$C_5$-Alkoxygruppe, einem Phosphorsäureamid, einer Amino-$C_1$-$C_5$-alkoxygruppe, einer $C_1$-$C_5$-Alkylamino-$C_1$-$C_5$-alkoxygruppe, einer Di-$C_1$-$C_5$-alkylamino-$C_1$-$C_5$-alkoxygruppe, einer Mercaptogruppe, einer $C_1$-$C_5$-Alkylthiogruppe, einer Aminogruppe, einer $C_1$-$C_5$-Alkylaminogruppe, einer Di-$C_{1}$-$C_5$-alkylaminogruppe, einer $C_1$-$C_5$-Alkylgruppe, einer Amino-$C_{1}$-$C_5$-alkylgruppe, einer Trifluormethylgruppe, einer $C_2$-$C_{6}$-Alkanoylgruppe, einer $C_2$-$C_6$-Alkanoylaminogruppe und einer Aminosäureacylaminogruppe ausgewählte Substituentengruppe ist, und X ein Wasserstoffatom oder eine Nitrilgruppe darstellt.

2. Antitumormittel nach Anspruch 1, worin das Stilbenderivat eine Verbindung mit einem cis-Stilbenskelett ist, welche in vitro eine inhibierende Aktivität auf die Tubulinpolymerisation und/oder eine Antitumoraktivität zeigt; und worin die Platinkoordinationsverbindung eine Verbindung mit Antitumoraktivität ist.

3. Antitumormittel nach Anspruch 1 oder 2, worin die Platinkoordinationsverbindung aus Cisplatin, Carboplatin und Nedaplatin ausgewählt ist.

4. Antitumormittel nach einem der vorstehenden Ansprüche, worin das Stilbenderivat durch die folgende allgemeine Formel (1) dargestellt ist und die Platinkoordinationsverbindung eine Verbindung mit Antitumoraktivität ist:

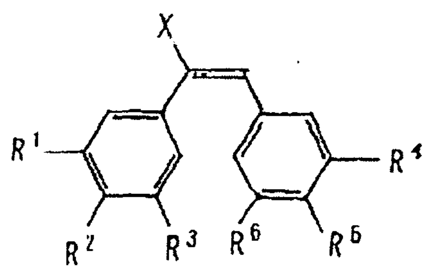

(1)

wobei die Verbindung in der Form eines Salzes sein kann; und worin $R^1$, $R^2$, $R^3$ und $R^5$ eine Methoxygruppe darstellen, $R^4$ einen beliebigen, unter einer Aminogruppe und einer Aminosäureacylaminogruppe ausgewählten Substituenten darstellt und $R^6$ und X ein Wasserstoffatom darstellen.

**5.** Antitumormittel nach Anspruch 4, worin das Stilbenderivat durch die folgende Strukturformel (3) dargestellt ist und die Platinkoordinationsverbindung eine Antitumoraktivität zeigt:

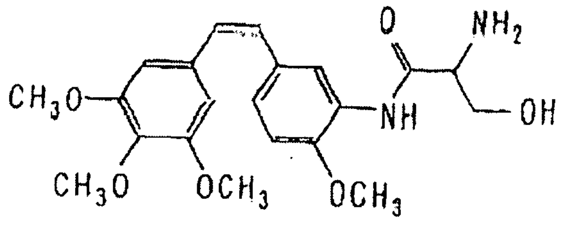

(3)

**6.** Antitumormittel nach einem der vorstehenden Ansprüche, worin die Platinkoordinationsverbindung Cisplatin ist und das Stilbenderivat eine Verbindung der folgenden Strukturformel (3) ist:

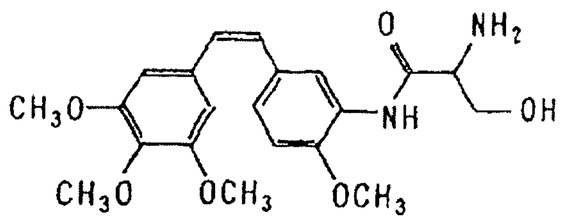

(3)

**7.** Verwendung eines Stilbenderivats der Formel (1) nach Anspruch 1 und einer Platinkoordinationsverbindung bei der Herstellung eines Antitumormittels oder eines Arzneimittels für die Behandlung, Unterdrückung oder Besserung eines Tumors.

**8.** Stilbenderivat der Formel (1) nach Anspruch 1 in Kombination mit einer Platinkoordinationsverbindung für die gleichzeitige oder getrennte Verwendung als Antitumormittel.

## Revendications

**1.** Agent antitumoral comprenant un dérivé du stilbène et un complexe de coordination du platine, et contenant éventuellement une ou plusieurs substances parmi les supports pharmaceutiquement acceptables, les diluants et d'autres substances nécessaires pour la préparation pharmaceutique où ledit dérivé du stilbène est au moins l'un des composés représentés par la formule générale (1) suivante :

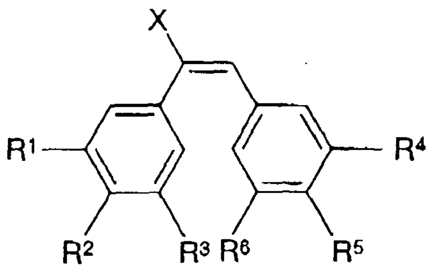

(1)

où le composé peut être sous forme de sel, d'hydrate ou de solvate ; où $R^1$, $R^2$ et $R^3$ sont indépendants les uns des autres et représentent chacun un groupe C1-C5 alcoxy, $R^4$, $R^5$ et $R^6$ sont indépendants les uns des autres et représentent chacun un groupe substituant quelconque d'un atome d'hydrogène, d'un atome d'halogène, d'un groupe nitro, d'un groupe C1-C5 alcoxy, d'un amide d'acide phosphorique, d'un groupe amino C1-C5 alcoxy, d'un groupe C1-C5 alkylamino C1-C5 alcoxy, d'un groupe di- C1-C5 alkylamino C1-C5 alcoxy, d'un groupe mercapto, d'un groupe C1-C5 alkylthio, d'un groupe amino, d'un groupe C1-C5 alkylamino, d'un groupe di-C1-C5 alkylamino, d'un groupe C1-C5 alkyle, d'un groupe amino C1-C5 alkyle, d'un groupe trifluorométhyle, d'un groupe C2-C6 alcanoyle, d'un groupe C2-C6 alcanoylamino et d'un groupe aminoacide acylamino, et X représente un atome d'hydrogène ou un groupe nitrile.

**2.** Agent antitumoral selon la revendication 1 où ledit dérivé du stilbène est un composé ayant un squelette de cis-stilbène présentant une activité inhibant la polymérisation de la tubuline in vitro et/ou une activité antitumorale ; et ledit composé de coordination du platine est un composé présentant une activité antitumorale.

**3.** Agent antitumoral selon la revendication 1 ou la revendication 2 où ledit composé de coordination du platine est choisi parmi le Cispiatine, le Carboplatine et le Nedaplatine.

**4.** Agent antitumoral selon l'une quelconque des revendications précédentes où ledit dérivé du stilbène est représenté par la formule générale (1) suivante, et ledit composé de coordination du platine est un composé présentant une activité antitumorale :

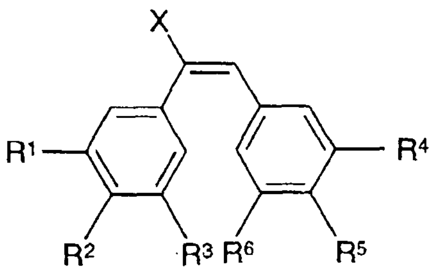

(1)

où ledit composé peut être sous forme de sel ; et où $R^1$, $R^2$, $R^3$ et $R^5$ désignent un groupe méthoxy, $R^4$ désigne un substituant quelconque d'un groupe amino et d'un groupe aminoacide acylamino, et $R^6$ et X désignent un atome d'hydrogène.

**5.** Agent antitumoral selon la revendication 4 où ledit dérivé du stilbène est représenté par la formule structurale (3) suivante, et ledit composé de coordination du platine est un composé présentant une activité antitumorale :

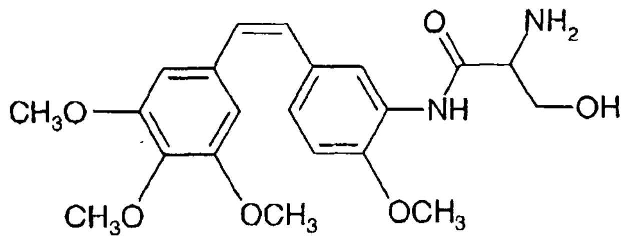

(3)

6. Agent antitumoral selon l'une quelconque des revendications précédentes où ledit composé de coordination du platine est le Cisplatine et ledit dérivé du stllbène est un composé représenté par la formule structure (3) suivante :

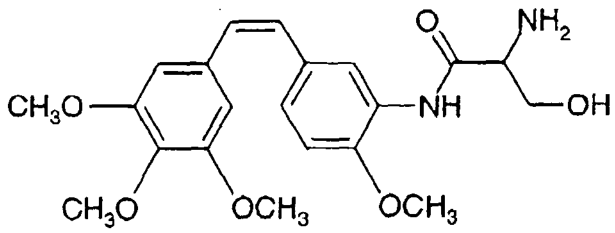

(3)

7. Utüisation d'un dérivé du stilbène de formule (1) selon la revendication 1 et d'un composé de coordination du platine dans la préparation d'un agent antitumoral ou d'un médicament pour le traitement, la suppression ou l'amélioration d'une tumeur.

8. Dérivé du stilbène de formule (1) selon la revendication 1 en combinaison avec un composé de coordination du platine pour l'utilisation simultanée ou séparée comme agent antitumoral.